# EUROPEAN PATENT SPECIFICATION

(11) **EP 1 952 785 B1**
(45) Date of publication and mention of the grant of the patent: **22.02.2012**
(21) Application number: 06817827.6
(22) Date of filing: 16.11.2006
(51) Int. Cl.: A61F 2/04, A61F 2/86, A61M 29/02

(54) **A CARDIA STENT**
KARDIA-STENT
ENDOPROTHESE CARDIALE

(30) Priority: 16.11.2005 CN 200510101177
(43) Date of publication of application: 06.08.2008
(73) Proprietor: Cheng, Yingsheng, Shanghai 200233 (CN); Zhou, Xing, Guangzhou, Guangdong 510060 (CN); Zhu, Ming, Haidian District Beijing 100088 (CN)
(72) Inventor: Cheng, Yingsheng, Shanghai 200233 (CN); Zhou, Xing, Guangzhou, Guangdong 510060 (CN); Zhu, Ming, Haidian District Beijing 100088 (CN)
(74) Representative: Lang, Christian
(86) International application number: PCT/CN2006/003078
(87) International publication number: WO 2007/056946

(56) References cited:
- WO-A1-2006/044640
- CN-A- 1 241 399
- CN-A- 1 765 339
- CN-Y- 2 595 394
- US-A1- 2004 102 855
- US-B1- 6 254 642
- US-B1- 6 302 917

## Description

### FIELD OF THE INVENTION

The present invention relates to a medical stent, and in particular to a cardia stent for expansion treatment on the narrow cardia of the oesophagus.

### BACKGROUND OF THE INVENTION

In the prior art, the narrow cardia is caused by variable reasons, for example, cardiac cancer, cardiac disorder, esophageal hiatal hernia, and other physical or chemistry damage, and will be caused by surgery treatment. Ming ZHU etc. had disclosed an intra-cavity support for medical in Chinese patent application No. 97200206.5 published on June 10, 1998 (CN2283469Y). But because the shape and position of the cardia, it needs to develop a cardia stent which could be suitable for the expansion treatment on the narrow cardia caused by variable reasons.

A conventional stent is described by US 2004/0102855 A1 which discloses an anti-reflux stent comprising an inner sleeve with a tapering distal end, a stent and a coating which connects the inner sleeve with the stent.

Starting from the cited prior art, it is the object of the present invention to provide an anti-reflux stent, which is configured to easily receive and output food through the stent which has improved anti-reflux property.

### SUMMARY OF THE INVENTION

This object is solved by the cardia stent according to claim 1. Advantageous improvements of the cardia stent are described by dependent claims.

The object of the present invention is further to provide a cardia stent for expansion treatment on the narrow cardia. The cardia stent has the advantages of: difficult to shift, matching with carida anatomy, high anti-reflux ability, easy operation, resisting the corrosion of gastric juice, and it can be used in expansion treatment on the narrow cardia caused by variable reasons.

A cardia stent according to the present invention is characterized in:
a. said cardia stent is woven by wires of shape memory NiTi alloy;
b. a drum-shaped locating port, disposed at the upper end of said cardia stent;
c. a trumpet-shaped locating port, disposed at the lower end of said cardia stent;
d. a supporting net tube having a ability of resisting constringency of the cardia, connected with said upper drum-shaped locating port and said lower trumpet- shaped locating port;
e. the remaining portions other than said drum-shaped locating port are coated with a membrane of medical flexible material that can be implanted into a human body; and
f. as least one anti-reflux valve, disposed at the connection part between said lower trumpet-shaped locating port and said supporting net tube, or disposed at the connection part between said upper drum-shaped locating port and said supporting net tube, or disposed at the inner part of said supporting net tube, wherein said anti-reflux valve is of a triple-petal structure protruding downward that is made from a membrane of medical flexible material that can be implanted into a human body.

According to the cardia stent of the present invention, the medical flexible material that can be implanted into a human body are selected from the following materials: silicon rubber, Polyurethane, Polytetrafluoroethylene, and Polypropylene.

Preferred cardia stent of the present invention comprises two anti-reflux valves, and the vertical projection between the split of three petals of first anti-reflux valve and the split of three petals of second anti-reflux valve is not overlap. That forms a cardia stent with a double-layer non-overlap anti-reflux device which can effectively increase the anti-reflux ability with better ventilation.

In a preferred embodiment, the cardia stent further comprising a retrieval string, installed in said upper drum-shaped locating port, which enables said upper drum-shaped locating port to be constringed. The retrieval string is used to regulate the position of the cardia stent and take the whole cardia stent out of the human body easily according the clinical situation.

The cardia carrier of the present invention utilizes a supporting net tube to match the upper locating port and the lower locating port, which has better positioning function and is difficult to shift. Wherein, the lower trumpet-shaped locating port and the supporting net tube are coated with a medical flexible material membrane to increase the ability of resisting the corrosion of gastric juice when the cardia carrier is inserted into the gastric juice. And a double-layer non-overlap anti-reflux device can effectively increase the anti-reflux ability with better ventilation. Further, a retrieval string is used to regulate the position of the cardia stent and take the whole cardia stent out of the human body easily according the clinical situation. The cardia stent has the advantages of: difficult to shift, matching with cardia anatomy, high anti-reflux ability, easy operation, resisting the corrosion of gastric juice, and it can be used in expansion treatment on the narrow cardia caused by variable reasons.

### BRIEF DESCRIPTION OF THE DRAWINGS

The invention is herein described, by way of example only, with reference to the accompanying drawings, wherein:
Fig. 1 is a perspective view illustrating the structure of a cardia stent with one anti-reflux valve according to the present invention;
Fig. 2 is a tridimensional graph illustrating the structure of the cardia stent of Fig. 1;
Fig. 3 is a perspective view illustrating the structure of a cardia stent with two anti-reflux valves according to the present invention;
Fig. 4 is a tridimensional graph illustrating the structure of the cardia stent of Fig. 3;
Fig. 5 is a perspective view illustrating a vertical projection between the split of three petals of first anti-reflux valve and the split of three petals of second anti-reflux valve;
Fig. 6 is a perspective view illustrating the structure of a cardia stent with one anti-reflux valve and a retrieval string according to the present invention;
Fig. 7 is a tridimensional graph illustrating the structure of the cardia stent of Fig. 6;
Fig. 8 is a perspective view illustrating the structure of a cardia stent with two anti-reflux valves and a retrieval string according to the present invention;
Fig. 9 is a tridimensional graph illustrating the structure of the cardia stent of Fig. 8; and
Fig. 10 is a perspective view illustrating the mechanism of the cardia stent according to the present invention.

The following list is a legend of the numbering of the application illustrations:
1 drum-shaped locating port
2 trumpet-shaped locating port
3 supporting net tube
4 membrane of medical flexible material that can be implanted into a human body
5 anti-reflux valve
6 retrieval string
7 split of three petals of the anti-reflux valve
8 wires of shape memory NiTi alloy
9 oesophagus
10 stomach
11 cardia stent according to the present invention

### DETAILED DESCRIPTION OF THE PREFERRED EMBODIMENT

### Example 1: A cardia stent with one anti-reflux valve

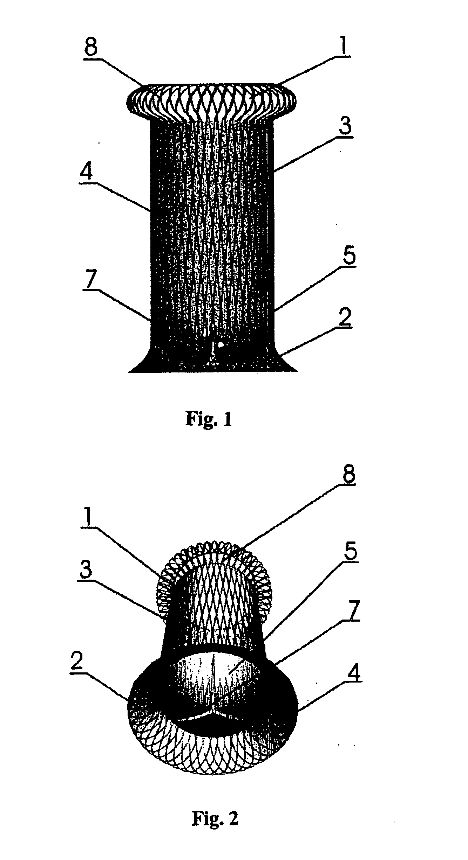

In this example, as illustrated in Fig.1 and FIG 2, a cardia stent is woven by wires 8 of shape memory NiTi alloy, said shape memory alloy has a recovery temperature between 25 °C and 33 °C and has a diameter between 0.2 mm and 0.25 mm. The cardia stent is finalized the design by heat treatment, and the finalized cardia stent comprises: a drum-shaped locating port 1, disposed at the upper end of said cardia stent; a trumpet-shaped locating port 2, disposed at the lower end of said cardia stent; and a supporting net tube 3 having a ability of resisting constringency of the cardia, connected with the upper drum-shaped locating port 1 and the lower trumpet-shaped locating port 2. The remaining portions other than said drum-shaped locating port 1 are coated with a membrane 4 of medical flexible material (for example, silicon rubber). We make a semi-spherical membrane of medical flexible material (for example, silicon rubber) between the lower trumpet- shaped locating port 2 and said supporting net tube 3, then shear the semi-spherical membrane into three petals, and an anti-reflux valve is formed. The anti-reflux valve 5 is of a triple-petal structure protruding downward. By washing, the cardia stent with one anti-reflux valve according to the present invention is obtained.

### Example 2: A cardia stent with two anti-reflux valves

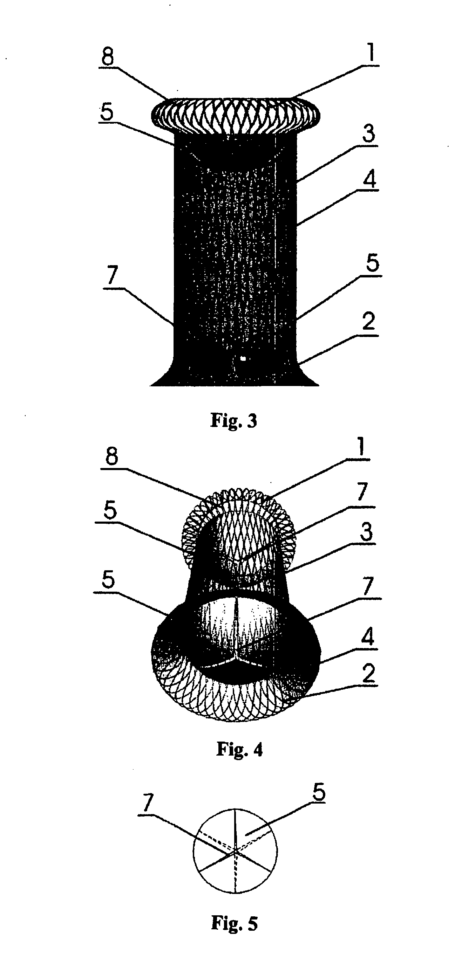

In this example, as illustrated in Fig. 3, Fig. 4 and Fig. 5, a cardia stent is made by the similar process showing in Example 1. But in this example, the cardia stent comprises two anti-reflux valves. The first anti-reflux valve is disposed at the connection part between the lower trumpet-shaped locating port 2 and the supporting net tube 3, and the second anti-reflux valve is disposed at the connection part between the upper drum-shaped locating port 1 and the supporting net tube 3. All of two anti-reflux valves 5 are of a triple-petal structure protruding downward. So the cardia stent with two anti-reflux valves according to the present invention is obtained.

### Example 3: A cardia stent with one anti-reflux valve and a retrieval string

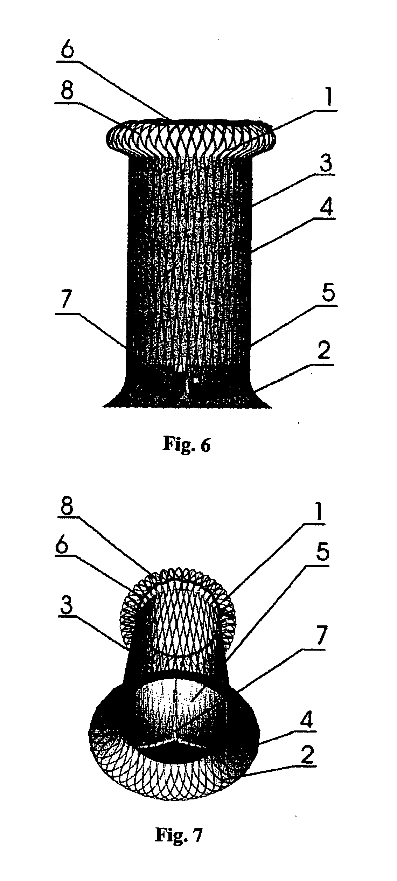

In this example, as illustrated in Fig. 6 and Fig. 7, a cardia stent is made by the similar process showing in Example 1. But in this example, we make a retrieval string (for example, surgical string) pass through the mesh of upper brim of the drum-shaped locating port 1 which is uncoated with a membrane. So the cardia stent with one anti-reflux valve and a retrieval string according to the present invention is obtained. When the retrieval string is tense, the drum-shaped locating port 1 is constringed, which cause the whole cardia stent to be constringed, then we could easily regulate the position of the cardia stent and take the whole cardia stent out of the human body.

### Example 4: A cardia stent with two anti-reflux valves and a retrieval string

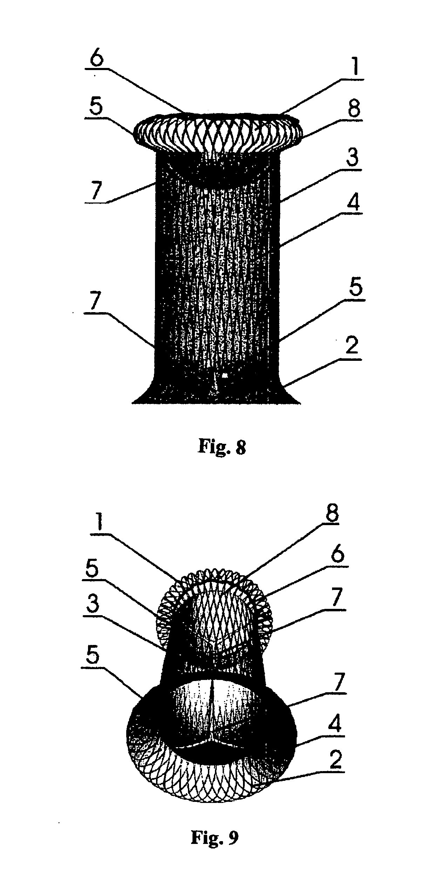

In this example, as illustrated in Fig. 8 and Fig. 9, a cardia stent is made by the similar process showing in Example 2 and Example 3. But in this example, we make a second anti-reflux valve 5. The second anti-reflux valve 5 is disposed at the connection part between the upper drum-shaped locating port 1 and the supporting net tube 3. All of two anti-reflux valves 5 are of a triple-petal structure protruding downward. So the cardia stent with two anti-reflux valves and a retrieval string according to the present invention is obtained.

In above-mentioned examples, the medical flexible material that can be implanted into a human body could be selected from the following materials: silicon rubber, Polyurethane, Polytetrafluoroethylene, and Polypropylene. And the recovery temperature and the diameter of the shape memory alloy could be adjusted according the actual situation. And the thickness and the position of the anti-reflux valve also could be adjusted according the clinical situation.

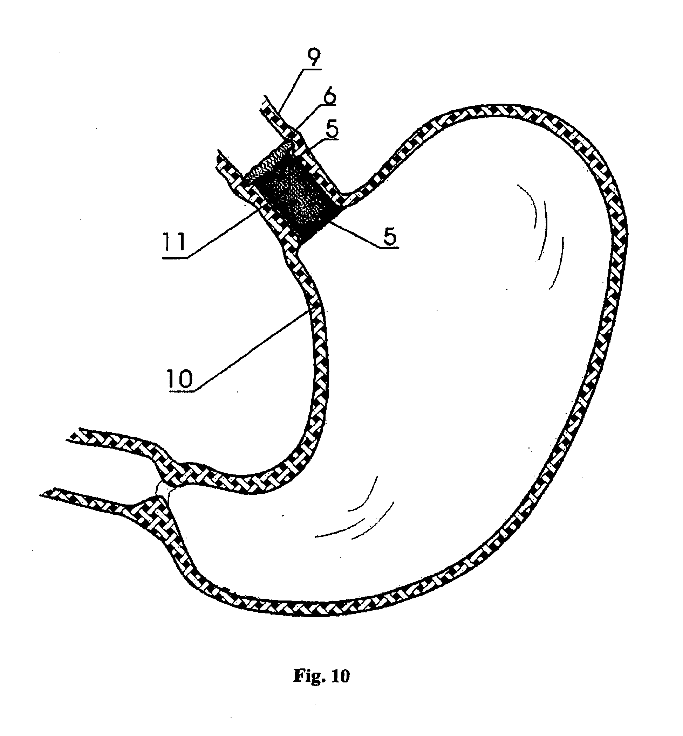

Referring to FIG. 10, when we embed the cardia stent according to the present invention, we first put the cardia stent 11 into a transporter, then let the cardia stent 11 release in the narrow position of the cardia, monitoring by the gastroscope or X-ray. The cardia stent will recover its pre-designed shape, the upper drum-shaped locating port 1 will be disposed in the oesophagus 9, and the lower trumpet- shaped locating port 2 will be disposed in the stomach 10. The anti-reflux valve 5 has the ability of resisting the corrosion of gastric juice. And the retrieval string is used to regulate the position of the cardia stent and take the whole cardia stent out of the human body easily.

## Claims

1. A cardia stent used on the narrow carida, and adapted to be placed between an oesophagus and a stomach, said cardia stent comprising:
a. woven wires (8) of shape memory NiTi alloy;
b. a drum-shaped locating port (1) comprising an inwardly projecting upper brim, disposed at an upper end of said cardia stent, said drum-shaped locating port (1) adapted for placement near a lower end of the oesophagus before the stomach;
c. a trumpet-shaped locating port (2), disposed at a lower end of said cardia stent, said trumpet-shaped locating port (2) adapted for placement in the stomach directly below the oesophagus;
d. a supporting net tube (3) having an ability of resisting constringency of said cardia, said supporting net tube (3) coupled between said upper drum-shaped locating port (1) and said lower trumpet- shaped locating port (2);
e. a membrane (4) of medical flexible material adapted to be implanted into a human body that coats remaining portions other than said drum-shaped locating port (1); and
f. at least one anti-reflux valve (5), disposed at a first connection part between said lower trumpet-shaped locating port (2) and said supporting net tube (3), or disposed at a second connection part between said upper drum-shaped locating port (1) and said supporting net tube (3), or disposed at an inner part of said supporting net tube (3), wherein said anti-reflux valve (5) is of a triple-petal structure protruding downward that is made from a semi-spherical membrane of medical flexible material and sheared into three petals, adapted to be implanted into a human body.

2. The cardia stent of claim 1, wherein said medical flexible material adapted to be implanted into a human body is selected from the group consisting of silicon rubber, Polyurethane, Polytetrafluoroethylene, and Polypropylene.

3. The cardia stent of claim 1, wherein said cardia stent comprising two anti-reflux valves (5), and a vertical projection of a split of three petals of first anti-reflux valve and a vertical projection of a split of three petals of second anti-reflux valve do not overlap.

4. The cardia stent of claim 1, wherein said cardia stent further comprising a retrieval string (6), installed in said drum-shaped locating port (1), which enables said drum-shaped locating port (1) to be constringed.

5. The cardia stent of claim 3, wherein said cardia stent further comprising a retrieval string (6), installed in said drum-shaped locating port (1), which enables said drum-shaped locating port (1) to be constringed.

## Patentansprüche

1. Cardiastent, der in der engen Cardia verwendet wird und der hergerichtet ist, zwischen einer Oesophagus und einem Magen platziert zu werden, wobei der Cardiastent umfasst:
a. gewebte Drähte (8) aus einer NiTi-Formgedächtnis- Legierung;
b. einem trommelförmigen Anordnungsanschluss(1), der einen nach innen gerichteten oberen Rand umfasst, der an einem oberen Ende des Cardiastent angeordnet ist, wobei der trommelförmige Anordnungsanschluss (1) für die Platzierung nahe dem unteren Ende der Oesophagus vor dem Magen ausgelegt ist;
c. einen trompetenförmigen Anordnungsanschluss (2), der an einem unteren Ende des Cardiastent angeordnet ist, wobei der trompetenförmige Anordnungsanschluss (2) für die Platzierung in dem Magen unmittelbar unterhalb der Oesophagus ausgelegt ist;
d. eine unterstützende Netzröhre (3), die die Eigenschaft aufweist, dem Zusammenziehen der Cardia zu widerstehen, wobei die unterstützende Netzröhre (3) zwischen dem oberen trommelförmigen Anordnungsanschluss (1) und dem unteren trompetenförmigen Anordnungsanschluss (2) mit diesen verbunden ist;
e. eine Membran (4) aus einem flexiblen medizinischen Material, die darauf ausgelegt ist, in einen menschlichen Körper eingesetzt zu werden, und die außer dem trommelförmigen Anordnungsanschluss (1) die verbleibenden Bereiche bedeckt; und
f. mindestens ein Anti-Rückflussventil (5), das in einem ersten Verbindungsbereich zwischen dem unteren trompetenförmigen Anordnungsanschluss (2) und der unterstützenden Netzröhre (3) oder in einem zweiten Verbindungsbereich zwischen dem oberen trommelförmigen Anordnungsanschluss (1) und der unterstützenden Netzröhre (3) oder an einem inneren Teil der unterstützenden Netzröhre (3) angeordnet ist, wobei das Anti-Rückflussventil (5) eine dreiblättrige Struktur aufweist, die sich nach unten erstreckt und die aus einer semi-sphärischen Membran hergestellt ist, welche aus flexiblem medizinischen Material hergestellt ist und in drei Blätter geschnitten ist, die hergerichtet sind, in den menschlichen Körper eingesetzt zu werden.

2. Cardiastent nach Anspruch 1, bei welchem das flexible medizinische Material, das angepasst ist, in einen menschlichen Körper eingesetzt zu werden, aus einer Gruppe ausgewählt ist, die Silikongummi, Polyurethan, Polytetrafluorethylen und Polypropylen umfasst.

3. Cardiastent nach Anspruch 1, wobei der Cardiastent zwei Anti-Rückflussventile (5) umfasst und wobei sich eine vertikale Projektion der Aufspaltung der drei Blätter des ersten Anti-Rückflussventils und eine vertikale Projektion der Aufspaltung der drei Blätter des zweiten Anti-Rückflussventils nicht überlappen.

4. Cardiastent nach Anspruch 1, wobei der Cardiastent ferner einen Strang (6) zur Wiedergewinnung umfasst, der in dem trommelförmigen Anordnungsanschluss (1) angebracht ist und es ermöglicht, den trommelförmigen Anordnungsanschluss (1) zusammenzuziehen.

5. Cardiastent nach Anspruch 3, wobei der Cardiastent ferner einen Strang (6) zur Wiedergewinnung umfasst, der in dem trommelförmigen Anordnungsanschluss (1) angebracht ist und der es ermöglicht, den trommelförmigen Anordnungsanschluss (1) zusammenzuziehen.

## Revendications

1. Endoprothèse de cardia utilisée sur le cardia étroit et adaptée pour être placée entre un oesophage et un estomac, ladite endoprothèse de cardia comprenant :
a. des fils tissés (8) avec mémoire de forme en alliage NiTi ;
b. une embouchure de localisation en forme de tambour (1) comprenant un brin supérieur qui fait saillie vers l'intérieur (1), disposée à une extrémité supérieure de ladite endoprothèse de cardia, ladite embouchure de localisation en forme de tambour (1) étant adaptée pour être placée près d'une extrémité inférieure de l'oesophage avant l'estomac ;
c. une embouchure de localisation en forme de trompette (2), disposée à une extrémité inférieure de ladite endoprothèse de cardia, ladite embouchure de localisation en forme de trompette (2) étant adaptée pour être placée dans l'estomac directement en dessous de l'oesophage ;
d. un tube de support en filet (3) étant capable de résister à la constringence dudit cardia, ledit tube de support en filet (3) étant couplé entre ladite embouchure supérieure de localisation en forme de tambour (1) et ladite embouchure inférieure de localisation en forme de trompette (2) ;
e. une membrane (4) en matériau médical flexible adaptée pour être implantée dans un corps humain qui enrobe les portions restantes autres que ladite embouchure de localisation en forme de tambour (1) et
f. au moins une valve anti-reflux (5) disposée sur une première partie de jonction entre ladite embouchure inférieure de localisation en forme de trompette (2) et ledit tube de support en filet (3) ou disposée sur une seconde partie de jonction entre ladite embouchure supérieure de localisation en forme de tambour (1) et ledit tube de support en filet (3) ou disposée sur une partie intérieure dudit tube de support en filet (3), ladite valve anti-reflux (5) étant en une structure en triple pétale faisant saillie vers le bas qui est faite en une membrane semi-sphérique en un matériau médical flexible et cisaillée en trois pétales, adaptés pour être implantés dans un corps humain.

2. Endoprothèse de cardia selon la revendication 1, ledit matériau médical flexible adapté pour être implanté dans un corps humain étant sélectionné dans le groupe comprenant le caoutchouc de silicone, le polyuréthane, le polytétrafluoroéthylène et le polypropylène.

3. Endoprothèse de cardia selon la revendication 1, ladite endoprothèse de cardia comprenant des valves anti-reflux (5) et une projection verticale d'une fraction de trois pétales de la première valve anti-reflux et une projection verticale d'une fraction de trois pétales de la ne se chevauchant pas.

4. Endoprothèse de cardia selon la revendication 1, ladite endoprothèse de cardia comprenant de plus un fil de retrait (6) installé dans ladite embouchure de localisation en forme de tambour (1) qui permet à ladite embouchure de localisation en forme de tambour (1) d'être constringée.

5. Endoprothèse de cardia selon la revendication 3, ladite endoprothèse de cardia comprenant de plus un fil de retrait (6), installé dans ladite embouchure de localisation en forme de tambour (1), qui perm et à ladite embouchure de localisation en forme de tambour (1) d'être constringée.
